# EUROPEAN PATENT SPECIFICATION

(11) **EP 3 677 303 B1**
(45) Date of publication and mention of the grant of the patent: **15.02.2023**
(21) Application number: 19150437.2
(22) Date of filing: 04.01.2019
(51) Int. Cl.: A61M 25/06, A61M 25/00, A61M 39/20

(54) **VASCULAR ACCESS CATHETER WITH SWABBABLE NEEDLE-FREE IV CONNECTOR**
GEFÄSSZUGANGSKATHETER MIT ABTUPFBAREM NADELFREIEM IV-VERBINDER
CATHÉTER D'ACCÈS VASCULAIRE AVEC CONNECTEUR IV DÉSINFECTABLE SANS AIGUILLE

(43) Date of publication of application: 08.07.2020
(73) Proprietor: Asset Medikal Tasarim Sanayi ve Ticaret A.S., Basaksehir-Istanbul (TR)
(72) Inventor: TÜYSÜZ, Mehmet, 34303 Istanbul (TR); BASARAN, Ahmet Reha, 34303 Istanbul (TR)
(74) Representative: Sögüt, Onur Ömer

(56) References cited:
- US-A- 6 145 688
- US-A1- 2010 292 673
- US-A1- 2015 374 969
- US-A1- 2016 089 530
- US-A1- 2017 120 014
- US-B1- 6 273 869

## Description

### Technical Field of the Invention

The present invention relates to a vascular access catheter for medical use. In particular, the present invention relates to such catheter combined with a swabbable needle-free intravenous connector.

### Background of the Invention

Patent applications US 2004/0193118 A1, US 2014/0180250 A1 and US 2015/265827 A1 relate to vascular access catheters for use in medicine.

US 5,674,206 A discloses a swabbable (or wipeable) valve or connector, used in an intravenous (iv) injection site. Swabbable iv connectors (or swabbable iv valves) have a swabbable portion (21) which can get exposed and thereby swabbed (or wiped) for cleaning before or after use.

US Pat. No. 8,377,040 B2 discloses a vascular access catheter in fluid communication with a swabbable iv connector, through a flexible hose. The flexible hose allows variations in direction and position of the iv connector relative to the cannula and therefore is open to cause discomfort by occasionally changing center of gravity of the combined catheter. The presence of flexible hose causes an extended size, an uneven distribution of weight which corresponds to discomfort in use; and furthermore, the hose brings a rather complex structure to the catheter which corresponds to increased production costs.

Said variations also correspond to limitation in ease of use of the catheter: position of the iv connector and that of any further fluid administering device attached to it may instantly or occasionally change because of gravity force exerted thereon.

US 2017/0120014 A1 relates to an integrated peripheral intravenous catheter having a low profile securement platform, a needle hub having an interior compartment and a paddle grip, and extension tube coupled to the catheter and stored within the interior compartment prior to catheterization. US 2015/0374969 A1 discloses a safety device for use with a medical implement having a body with an inlet end portion; the safety device comprises a bracket for mounting on the body of the medical implement, a cap supported by the bracket and sized to fit over and seal the inlet end portion of the medical implement in a safety position wherein the cap covers the medical implement; an elongate shaft having a longitudinal axis, the shaft being secured to the cap and being slidably and pivotably supported by the bracket so that the cap can be raised away from the inlet end portion of the medical implement and be pivoted about the longitudinal axis of the shaft away from the inlet end portion; and a biasing means for biasing the cap to the safety position. US Patent nr. 6,145,688 relates to an injection molded assembly forming a sealing device for a threaded or non-threaded container using a double cap concept with pressure responsive convex sealing; and a wiping mechanism, a one piece construction, a tamper resistant construction, a limited air exchange construction while allowing easy access to fluid contents, a sterile air venting and fillter construction, a one piece tamper evident closure with a tethered container, and a frangible thether means molder with the formation of the sealing device. US 6,273,869 B1 disclosed a hollow cannula made with a smooth tip to avoid sharp corners on an inside or outside diameter in order to avoid scraping or scoring of a slit or pre-pierced septum during passage through the septum, and the cannula may be employed in swabbable valve connectors.

Flexible elongate connections like hose correspond to a level of dishevelment and compactness is not available in devices with such parts.

### Objects of the Invention

Primary object of the present invention is to overcome the abovementioned shortcomings of the prior art.

Another object of the present invention is to provide a simplified and compact vascular access catheter assembly provided with a swabbable iv connector.

Another object of the present invention is to provide a vascular access catheter assembly with enhanced safety.

Another object of the present invention is to provide a vascular access catheter assembly with enhanced ease of use.

A further object of the present invention is to provide a vascular access catheter assembly offering enhanced comfort to patients.

### Summary of the Invention

The present invention proposes a vascular access catheter assembly including a main body, a needle secured to a needle hub which is releasably connected to the main body, a cannula attached to the main body and adapted to coaxially surround the needle, and a swabbable intravenous valve securely attached to the main body; such that at a first state of the assembly, the intravenous valve is positioned substantially around a retraction axis of the needle. The valve includes a core and the assembly comprises a cap which is releasably connected to the main body. The needle hub is secured to the cap, or integral with the cap. The valve is adapted by its shape, size and position relative to the main body, to be movable relative to the main body along the retraction axis towards the cannula at bringing the assembly from its first state to a second state by moving the needle hub towards the main body until a tip of the needle is exposed from the cannula. The valve includes a gap between the core and the cap adapted to be filled by moving the cap along with the valve towards the core at bringing the assembly from the first state to the second state.

### Brief Description of the Figures

The figures, whose brief explanation is herewith provided, are solely intended for providing a better understanding of the present invention and are as such not intended to define the scope of protection or the context in which said scope is to be interpreted in the absence of the description.
Fig.1 is a side view of an exemplary first embodiment of the catheter assembly according to the present invention, at a first state thereof.
Fig.2 is B-B section view of the exemplary first embodiment of the assembly shown in the Fig.1 at the first state, emphasizing an exemplary positioning of a swabbable intravenous valve in said assembly.
Fig.3 is a perspective view of the exemplary first embodiment of the assembly shown in the Fig.1, at a second state.
Fig.4 is a side view of the exemplary first embodiment of the assembly at the second state.
Fig.5 is C-C section view of the exemplary first embodiment of the assembly shown in the Fig.4 at the second state, emphasizing an exemplary positioning of a swabbable intravenous valve in said assembly and exposition of the tip of the needle in the second state.
Fig.6 is another perspective view of the exemplary first embodiment of the catheter assembly according to the present invention, emphasizing exposition of the tip of the needle at the second state.
Fig.7 is a side view of the exemplary first embodiment of the assembly shown in the Fig.6, at the second state.
Fig.8 is E-E section view of the exemplary first embodiment of the assembly shown in the Fig.7 at the second state, an exemplary positioning of a swabbable intravenous valve in said assembly and exposition of the tip of the needle in the second state.
Fig.9 shows side view of the exemplary first embodiment of the assembly according to the present invention at transition from the second state to a third state thereof, in which the tip of the needle is retracted into the cannula at distancing of the cap (40) from the main body, and the swabbable portion (21) of the valve (20) is exposed simultaneously.
Fig.10 is the F-F section view of the exemplary first embodiment of the assembly shown in Fig.9 at transition from the second state to the third state.
Fig.11 a side view of the exemplary first embodiment of the assembly at the third state wherein the needle is removed and the swabbable portion of the valve is exposed.
Fig.12 is D-D section view of the exemplary first embodiment of the assembly shown in the Fig.11, emphasizing an exemplary exposal of the swabbable portion of the valve at the third state.
Fig.13 is a perspective view of an exemplary second embodiment of the catheter assembly according to the present invention, at the first state thereof.
Fig.14 is a side view of the exemplary second embodiment of the assembly shown in the Fig.13, at the first state.
Fig.15 is A-A section view of the exemplary second embodiment of the assembly shown in the Fig.14 at the first state, showing an exemplary holder with a weakened portion between a needle hub and a main body of said assembly.
Fig.16 is s section view along the retraction axis of an exemplary first embodiment of the assembly, in which the valve is movable relative to the main body at bringing the assembly from the first position to the second position thereof.

### Detailed Description of the Invention

Referring now the figures outlined before, the present invention proposes a vascular access catheter assembly (1) including a main body (10) and a needle (11) secured to a needle hub (12). The catheter assembly (1) further comprises a cannula (50) which is attached to the main body (10) and which is adapted to coaxially surround the needle (11). The catheter assembly (1) further comprises a swabbable intravenous valve (20) (hereinafter abbreviated as valve). The valve (20) is securely attached to the main body (10), such that at a first state of the assembly (1) the intravenous valve (20) is positioned substantially around a retraction axis (X) of the needle (11).

The first state corresponds to an original state of the assembly (1) before preparation thereof for being mounted to a patient.

The assembly is compact and extremely reliable by having minimized distance between the valve (20) and the main body (10), without necessitating any unduly elongate fluid communication means therebetween.

This set of features eliminates movement of the valve (20) relative to the main body (10) (especially when compared to a case where the valve is attached on an end of a flexible hose), thereby eliminating the occurrence of pain related to variations in the position of the center of gravity related to combined parts of the assembly (1), and accordingly offers enhanced comfort to patients. In other words, the assembly according to the present invention has an enhanced rigidity between the main body (10) (which includes cannula (50)) and the valve (20), thereby the present invention provides an enhanced comfort to patients, in particular to pediatric patients by eliminating any dangling of the valve (20) on the main body (10).

This set of features further offers an enhanced ease of use: the valve (20) maintains its position and direction relative to the main body (10) (and also relative to where the assembly (1) is mounted to a tissue of a patient when the assembly is in use), instead of being on an end of a flexible hose.

Furthermore the present invention minimizes the costs for obtaining a compact assembly, by eliminating the necessity of assembling a commercially available valve and commercially available cannula as separate articles.

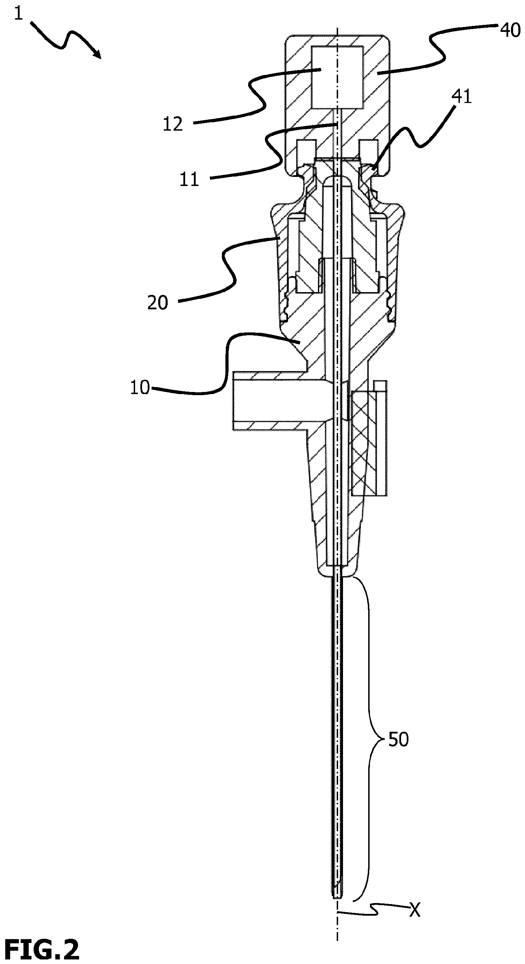

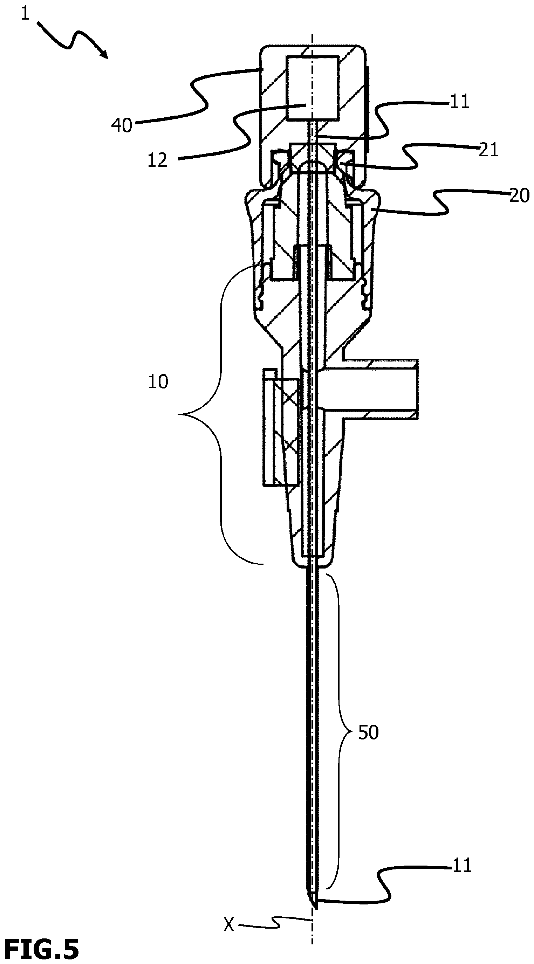

The valve (20) being securely attached (i.e. fixed directly, in an unmediated fashion) to the main body (10) provides enhanced compactness to the assembly (1). The compactness is even further enhanced in an embodiment where the main body and the valve are at least partly interbedded, e.g. as shown in Fig.2, Fig.5, Fig.8, Fig.10, Fig.12 and Fig.15.

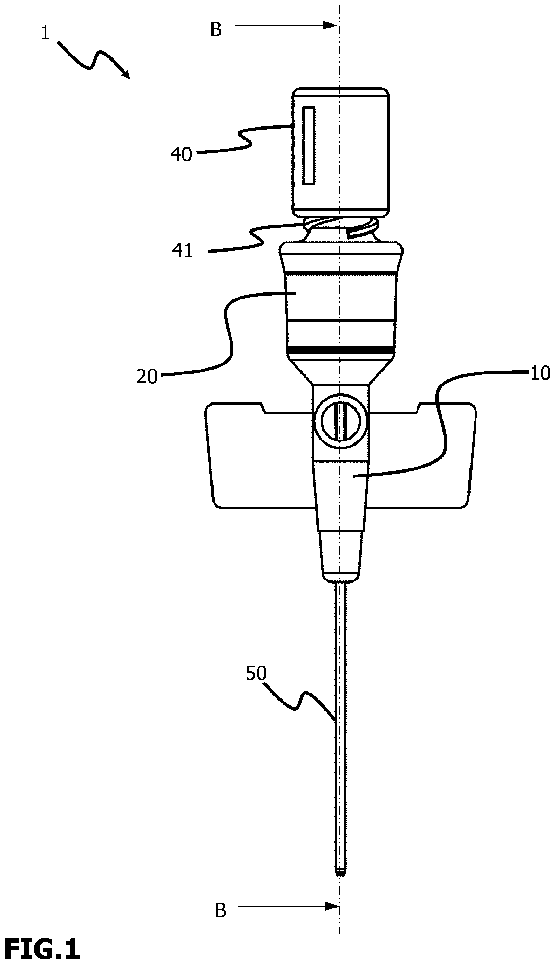

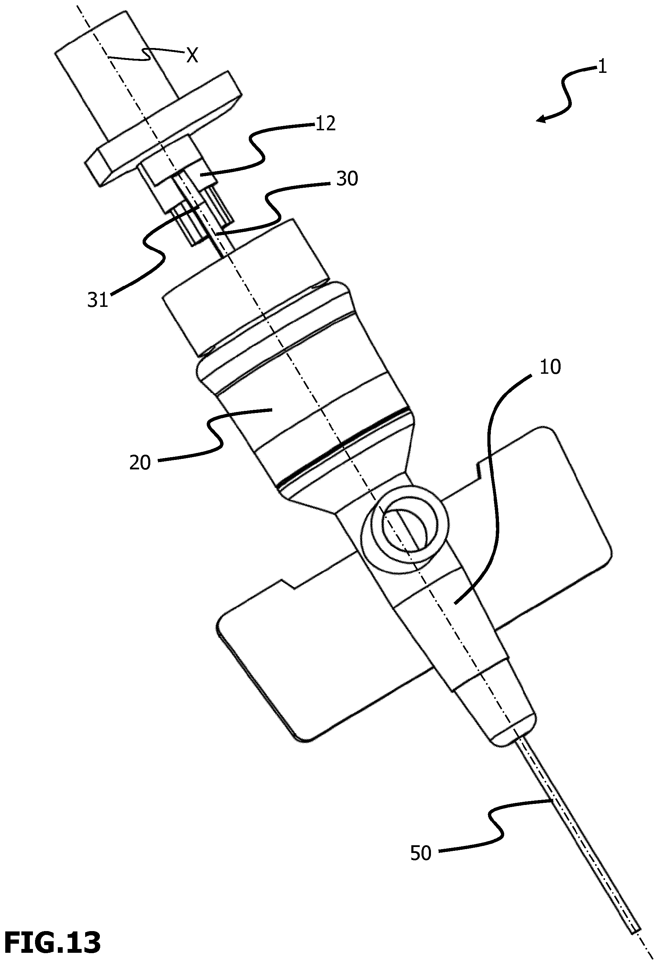

At the first state of the assembly (1), the needle (11) is substantially surrounded by the cannula (50) for prevention of accidental penetration of a tip of the needle (11) distal to the needle hub (12). Such first state is exemplified in Fig.1 and Fig.2 for a first embodiment; and in Fig.13, Fig.14 and Fig.15 for a second embodiment of the assembly (1) according to the present invention. The term "tip" is hereby used for naming an end of the needle (11) distal to the needle hub (12).

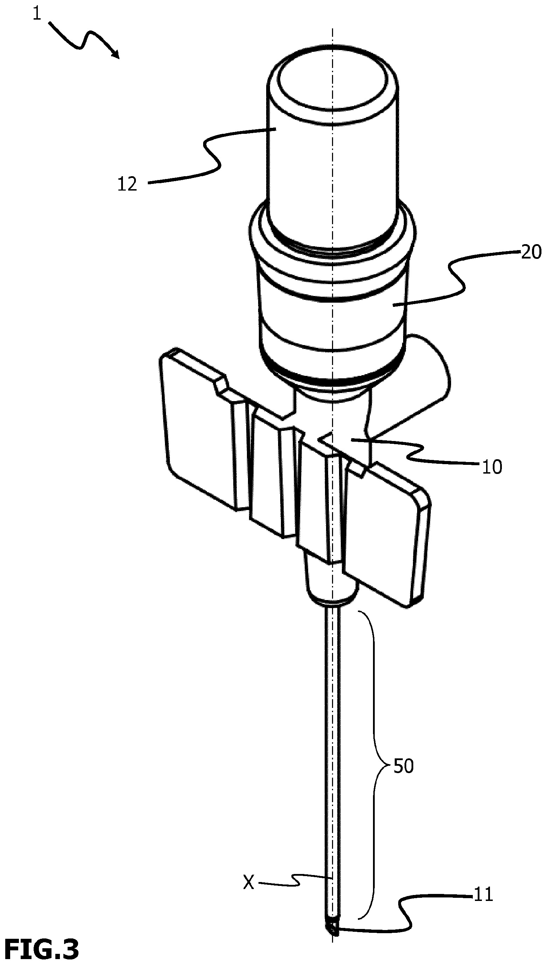

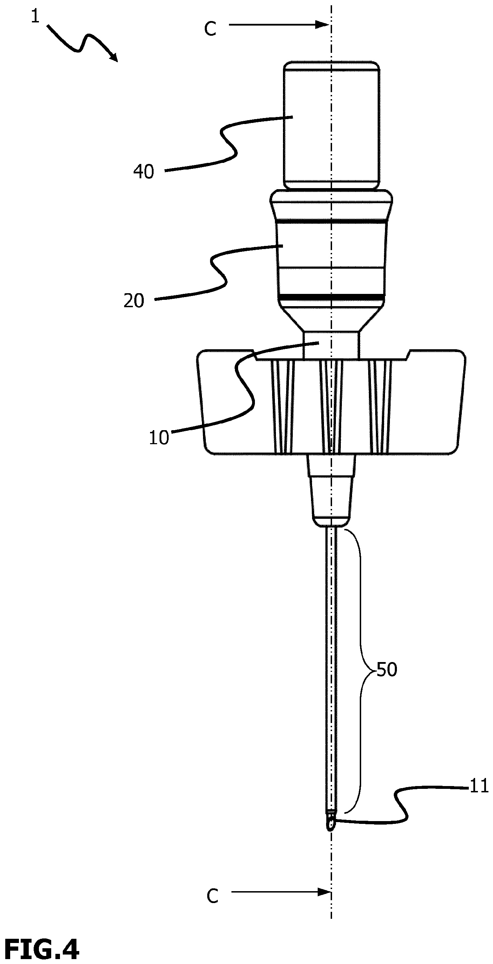

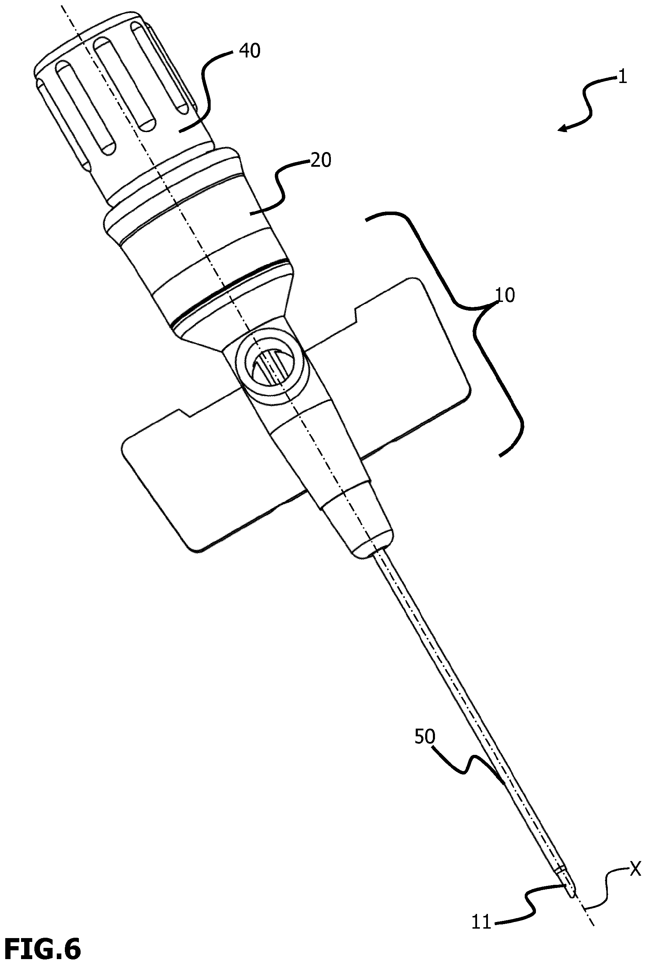

To prepare the assembly (1) for mounting to a patient, it is to be brought into a second state thereof, by moving the needle hub (12) towards the main body (10) until said tip of the needle (11) is exposed from the cannula (50). Such second state is visually exemplified in Fig.3, Fig.4, Fig.5, Fig.6, Fig.7 and Fig.8 for the first embodiment; and the same result is evidently available for the second embodiment of the assembly (1) according to the present invention.

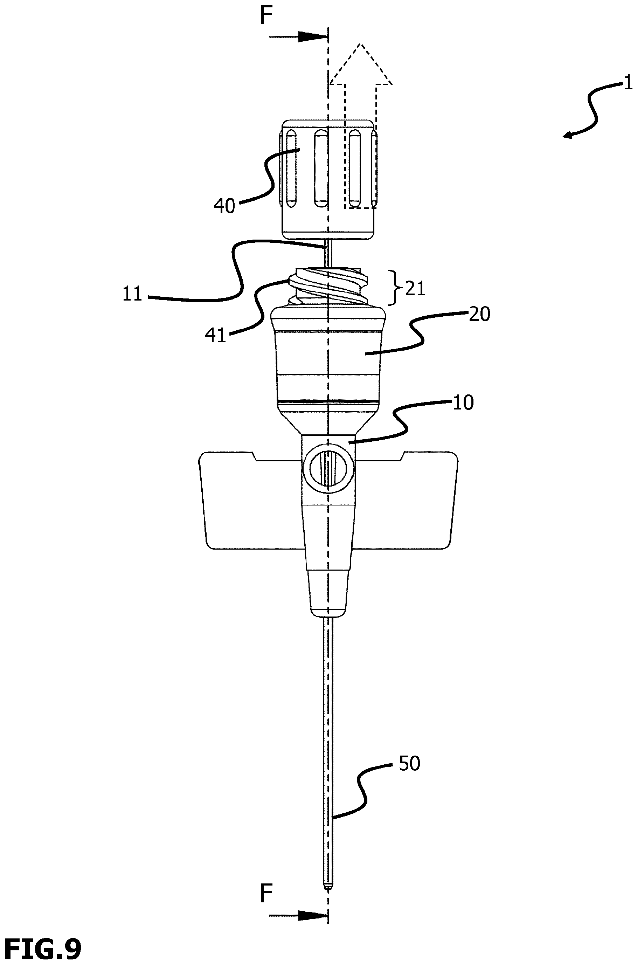

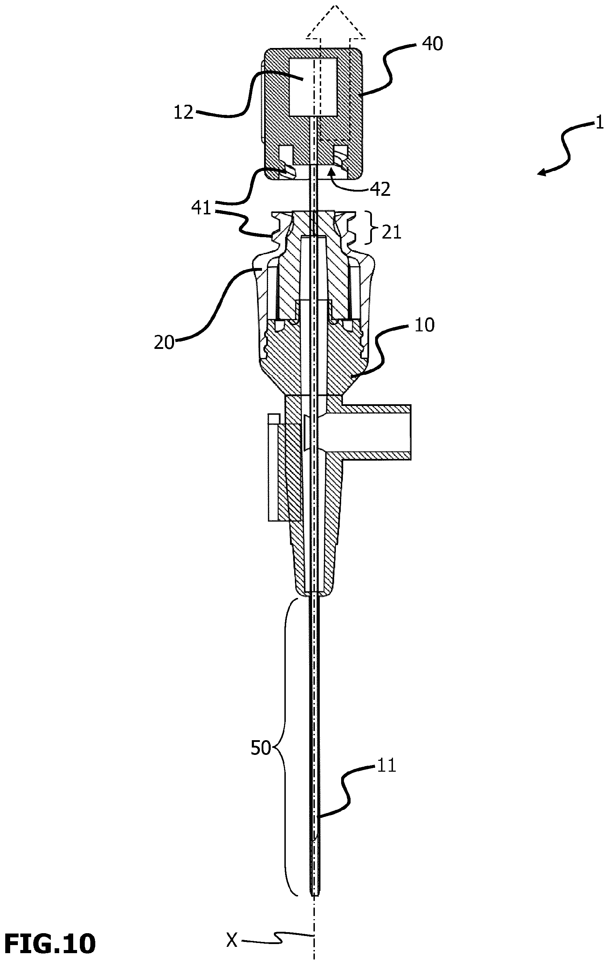

Upon mounting the assembly (1) to a patient, the needle (11) is to be removed by distancing the same along the retraction axis (X), from the cannula (50), main body (10) and valve (20). This phase, which is visually exemplified in the Fig.9 and Fig.10, corresponds to a transition from the second state to a third state of the assembly (1).

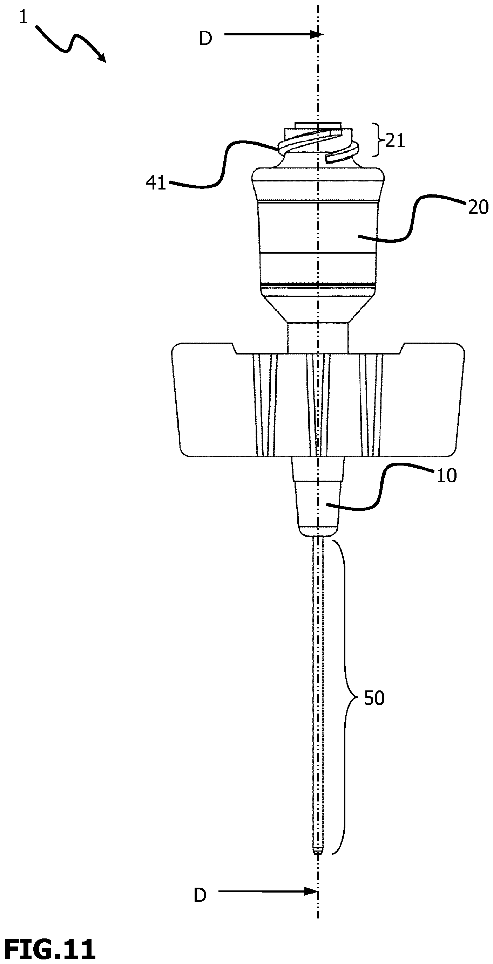

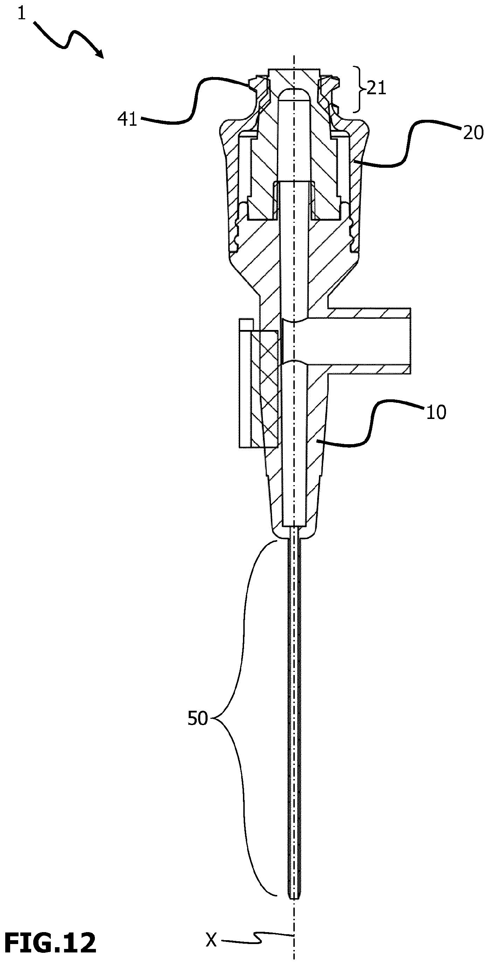

In the third state, the assembly (1) is ready to use for fluid transfer through the valve (20), main body (10) and cannula (50); and a swabbable portion (21) of the valve (20) is exposed. Such third state is visually exemplified in Fig.11 and Fig.12 for the first embodiment; and the same result is evidently available for the second embodiment of the assembly (1) according to the present invention. For the sake of simplicity, the removed parts (e.g. the needle, the needle hub, or the cap when applicable) are not shown in Fig.11 and Fig.12.

The needle hub (12) being releasably connected to the main body (10), enables that the needle (11) stays in place (i.e. substantially inside the cannula) until losing the releasable connection between the hub (12) and the main body (13) by intentional removal of the needle (11) from the main body (10) as in bringing the assembly (1) to the third state thereof.

In a first exemplary embodiment according to the present invention, the assembly (1) comprises a cap (40) releasably connected to the main body (10). The needle hub (40) is secured to the cap (40). Said cap (40) can have a size and shape to at least partly cover an end of the needle hub (12) distal to the canulla (50). The cap (40) is thereby arranged to prevent accidental distancing of the needle hub (12) from the main body (10). Thus, accidental removal of the needle (11) from the catheter assembly (1) is prevented by the cap (40). Fig.1 to Fig.12 present visual exemplifications to the first embodiment: the first state is shown in Fig.1 and Fig.2, the second state is shown in Fig.3 to Fig.8, transition from the second state to the third state is shown in Fig.9 and Fig.10, and third state is shown in Fig.11 and Fig.12.

In a version of this embodiment, the cap (40) can be connected to the main body (10) over a tamper-evident connection (not shown). This can be achieved e.g. by a cap (40) connected to the main body (10) over one or more bridges adapted to break when the cap (40) is moved relative to the main body (40) at a transition from the first state to another state (i.e. second state or third state).

In a possible embodiment according to the present invention, the cap (40) and the main body (10) may have screws adapted to engage with each other. This enables reversible removability of the cap (40) or needle hub (12) from the body (10). For instance, a cavity (42) of the cap (40) and an outer surface of the valve (20) substantially circumfering the swabbable portion (21) may be provided with said screws as visually exemplified in Fig.2, Fig.5, Fig.8, and in particular in Fig.10 to Fig.12.

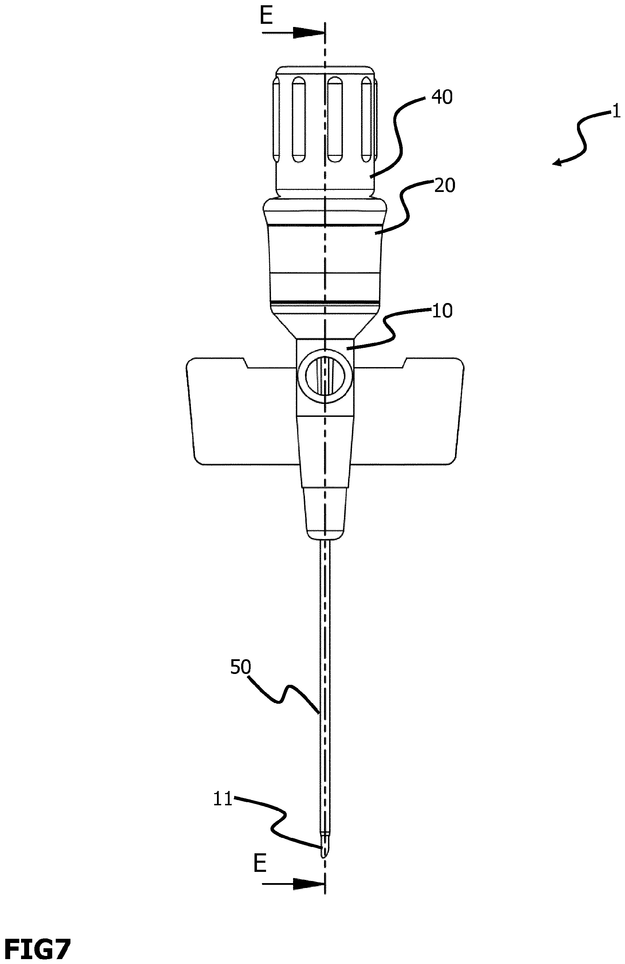

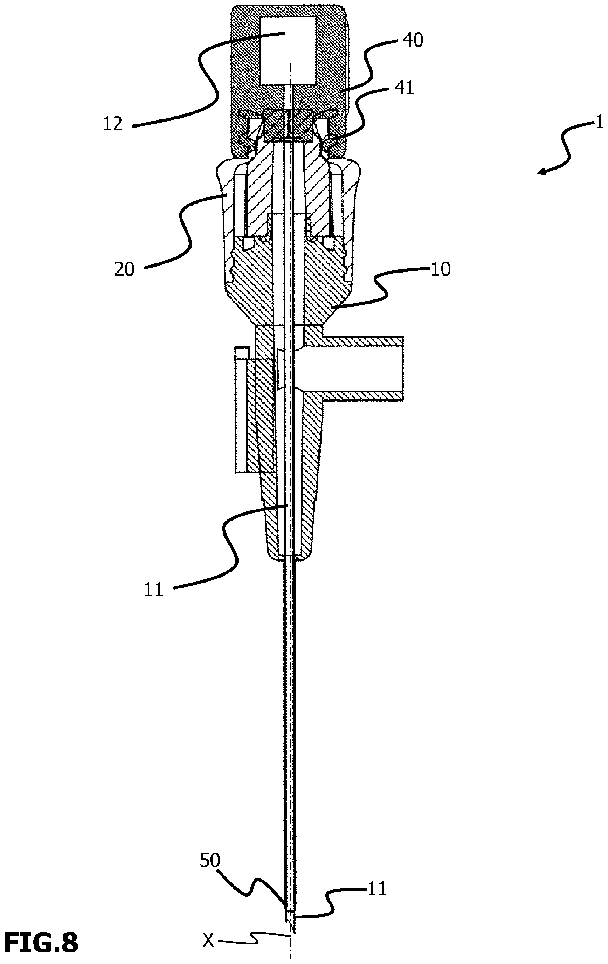

In a possible version of this embodiment, the cap (40) can be arranged by its shape and size to further receive a swabbable portion (21) of the valve (20), and preferably thereby substantially isolate said swabbable portion (21) from the surroundings and protect the same from contamination. In this version, the cap (40) can substantially receive the swabbable portion (21) into its cavity (42) in particular in the second state of the assembly (1). The main body (10) can have a shape and size adapted to be substantially interbedded with the valve (20), such that the swabbable portion (21) has a minimized distance to the canulla (50) thereby the assembly (1) is rendered more compact. Such positioning of the swabbable portion (21) where the same is substantially isolated from the surroundings by means of the cap (40) in the second state, is exemplified in Fig.8 which corresponds to the E-E section view of the assembly shown in the Fig.7.

Fig.9 shows side view of the exemplary first embodiment of the assembly according to the present invention at transition from the second state to the third state, in which the tip of the needle is retracted into the cannula at distancing of the needle hub (12) from the main body (10), upon which a simultaneous exposure of the swabbable portion (21) takes place.

Once the needle (11) is completely removed from the assembly (1) (e.g. as in the third state), the swabbable portion (21) of the valve (20) gets exposed to the surroundings and also ready for use in e.g. swabbing or administering fluids to a patient when the assembly is positioned for intravenous cannulation. The retraction of the needle at the third state is emphasized with a bold arrow in Fig.9 and in Fig.10 showing the F-F section view of the assembly shown in Fig.9. The swabbable portion (21) can be e.g. a male luer connection as exemplified in the accompanying drawings.

The needle hub (12) can be secured to the cavity (42) of the cap (40), and thereby arranged to conduct a movement of the cap (40) to the needle (hub (12) in case where the cap (40) is pressed towards the main body (10) so that a tip of the needle (11) distal to the cap (40) is extracted and exposed to pierce tissue of a patient for reaching her bloodstream.

In a further version according to the present invention, the cavity (42) of the cap (40) includes a cleaning means (not shown) including a cleaning liquid used for cleaning or sterilization of swabbable iv valves. In the second state of such particular embodiment, the swabbable portion (21) can be swabbed at bringing the assembly (1) into its second state. Swabbing at the second state provides enhanced hygiene or sterilization, thus enables a safe use of the assembly directly upon attaching the same to a patient. This feature further serves for temporary protection of the swabbable portion (21) from microbial growth even after contamination by microorganisms upon detachment of the cap (40) from the main body (10). The cleaning means can be exemplified as a material wetted or soaked with such cleaning liquid.

In a possible embodiment, the cap (40) can include a substantially resilient material, for instance at its surfaces which can be in contact with the swabbable portion (21) at the first state; such that the cavity (42) is sealed against leakage of fluids between the cavity (42) and surroundings in the first state of the assembly (1). The present embodiment substantially blocks microbial contamination of the swabbable portion (21) from the surroundings. Said fluid can be a cleaning liquid as mentioned above. In this case, the present embodiment enables maintenance of the cleaning liquid inside the cavity until the use of the assembly (1), thereby ensuring the hygiene or sterilization of the swabbable portion (21) without necessitating further swabbing upon exposal thereof at the third state.

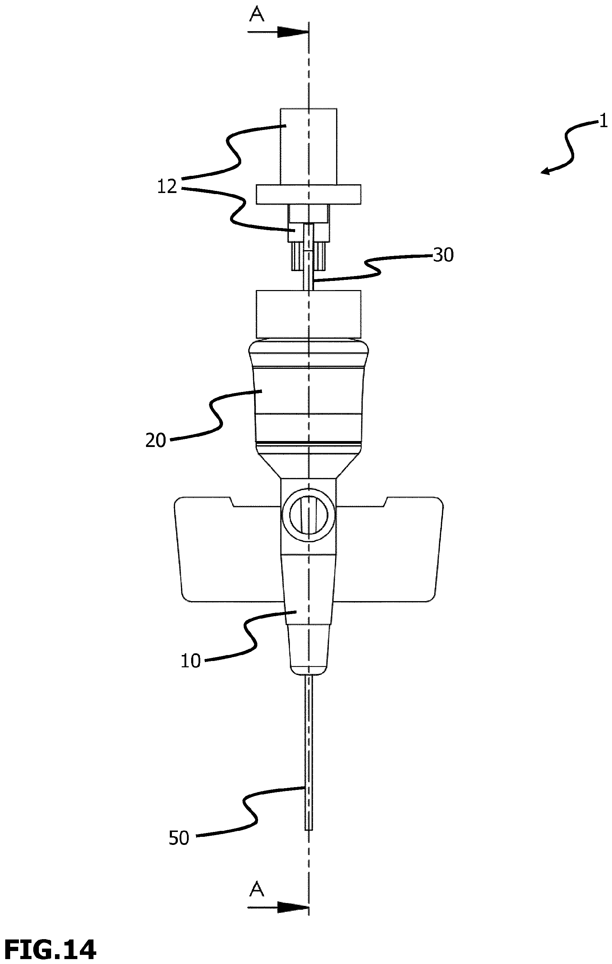

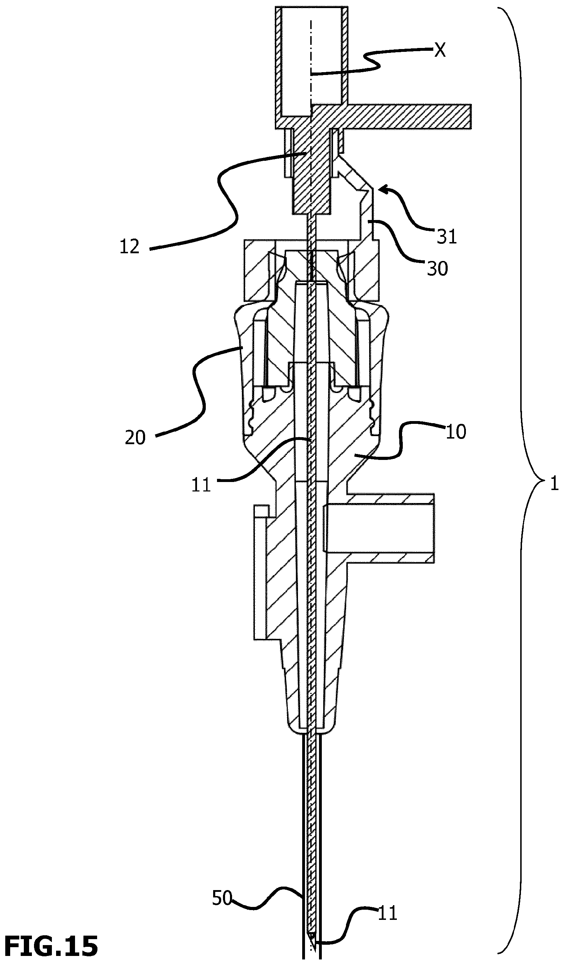

Perspective view of a second exemplary embodiment of the assembly (1) according to the present invention is presented in the Fig.13. A side view of the assembly exemplified in the Fig.1 is shown in the Fig.14, and A-A section thereof is exemplified in the Fig.15.

In the exemplary second embodiment according to the present invention, the assembly further comprises a holder (30) providing said releasable connection between the needle hub (12) and the main body (10) by having a first end secured to the needle hub (12) and a second end secured to the main body. The holder (30) is adapted to break when bent or forced, e.g. along or around the retraction axis (X). Thereby the holder (30) is arranged to break upon moving the needle hub (12) (e.g. towards the main body (10)). The needle (11) can be removed by pulling out of the cannula (50) by distancing the needle hub (12) from the main body (10).

The first end of the holder (30) can be directly fixed to the needle hub (12) as depicted in Fig.13 to Fig.15. In the case where the assembly (1) includes a cap (40), the first end of the holder (30) can alternatively be fixed to the cap (40).

The holder (30) can be provided with a weakened portion (31) as visually exemplified in Fig. 15, for facilitation of said rupture.

The holder (30) can be formed from a substantially inflexible material, such as a thermoset resin; with which a breaking behavior as described above is more easily achievable. Deformation of the holder (30) by breaking it at its weakened portion (31) further provides tamper evidence to the assembly (31), thereby providing enhanced safety to the assembly (1).

The weakened portion (31) can be located between the first end and the second end, as exemplified in the Fig. 15.

Alternatively, the weakened portion (31) can be located in vicinity of the second end of the holder (30), or at second end of the holder (30). This embodiment allows detachment of the holder (30) from the main body (10) as a whole, thereby allowing improved availability of the valve (20) for cleaning after removal of the holder (30).

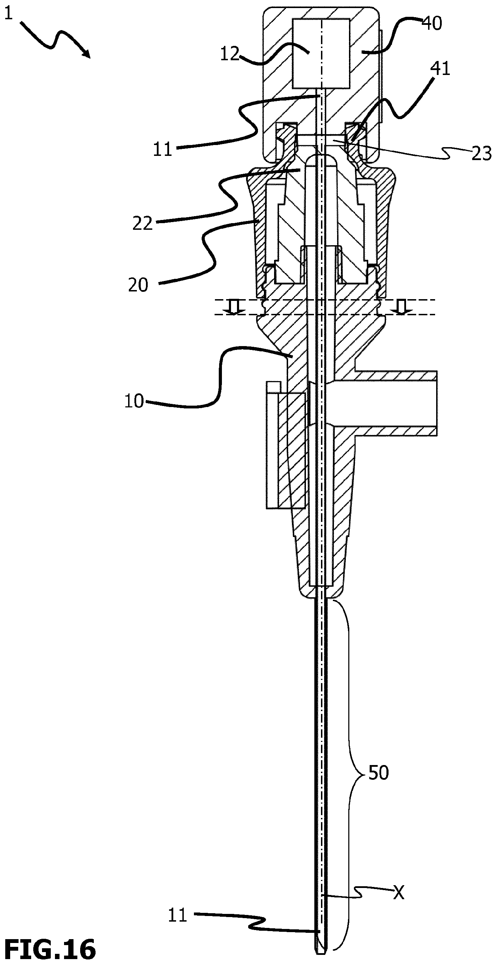

The valve (20) is adapted by its shape, size and position relative to the main body (10), to be movable (preferably with a substantially irreversible movement) relative to the main body (10) along the retraction axis (X) towards the cannula (50) at bringing the assembly (1) from its first state to second state. In other words, relative positioning between the valve (20) and the main body (10) can be adapted such that the valve (20) is to be moved relative to the main body (10) towards the cannula (50) along the retraction axis (X) and preferably to thereby bring the assembly (1) from its first state to second state. Here, a gap (23) is present between the core (22) and the cap (40), which is adapted to be filled by moving the cap (40) along with the valve (20) towards the main body (10) (thus towards the core (22)) at bringing the assembly (1) from the first state to thesecond state thereof. According to the invention, which is exemplified in the Fig. 16 at its first state, allows that a cap-side of a core (22) of the valve (20) or of its swabbable portion (21) can be kept at a minimized pressure until the assembly (1) is used. In Fig.16, the movement of the valve (20) relative to the main body (10) is symbolized with bold arrows. This feature protects the core (22) against deformation due to prolonged pressure exerted by the needle hub (12) or by an inner surface of the cap (40), in particular in case where the core (22) is flexible or elastic (e.g. when the core 22 is made of a polysiloxane material).

Thus the following objects are achieved by the present invention:
- the abovementioned shortcomings of the prior art are overcome,
- a compact vascular access catheter assembly with enhanced safety, ease of use, decreased costs and enhanced user comfort is provided.

### List of reference numerals:

- 1: catheter assembly (or assembly)
- 10: main body
- 11: needle
- 12: needle hub
- 20: valve
- 21: swabbable portion
- 22: core
- 23: gap
- 30: holder
- 31: weakened portion
- 40: cap
- 41: screw threads
- 42: cavity
- 50: cannula
- X: retraction axis

## Claims

1. A vascular access catheter assembly (1) including:
- a main body (10),
- a needle (11) secured to a needle hub (12) which is releasably connected to the main body (10),
- a cannula (50) which is attached to the main body (10) and adapted to coaxially surround the needle (11), and
- a swabbable intravenous valve (20) securely attached to the main body (10), such that at a first state of the assembly (1), the intravenous valve (20) is positioned substantially around a retraction axis (X) of the needle (11);
**characterized by**
- the valve (20) including a core (22);
wherein
the assembly comprises a cap (40) which is releasably connected to the main body (10), and the needle hub (12) is secured to the cap (40), or integral with the cap (40);
the valve (20) is adapted by its shape, size and position relative to the main body (10), to be movable relative to the main body (10) along the retraction axis (X) towards the cannula (50) at bringing the assembly (1) from its first state to a second state by moving the needle hub (12) towards the main body (10) until a tip of the needle (11) is exposed from the cannula (50); and
the valve (20) includes a gap (23) between the core (22) and the cap (40) adapted to be filled by moving the cap (40) along with the valve (20) towards the core (22) at bringing the assembly from the first state to the second state.

2. The assembly according to the claim 1wherein the cap (40) has a size and shape to at least partly cover an end of the needle hub (12) distal to the canulla (10).

3. The assembly according to any one of the claims 1 or 2, wherein the cap (40) is connected to the main body (10) over a tamper-evident connection.

4. The assembly according to any of the claims 1 to 3, wherein the cap (40) and the main body (10) have screw threads adapted to releasably engage with each other.

5. The assembly according to the claim 4, the cap (40) is arranged by its shape and size to further receive a swabbable portion (21) of the valve (20) into an cavity (42) thereof, so that said swabbable portion (21) is substantially isolated from surroundings in the first state of the assembly (1).

6. The assembly according to the claim 5, wherein said cavity (42) of the cap (40) includes a cleaning means comprising a cleaning liquid which is suitable for cleaning or sterilization of swabbable intravenous valves.

7. The assembly according to the claim 6, wherein the needle hub (12) is secured to the cavity (42) of the cap (40).

8. The assembly according to any one of the claims 1 to 7, wherein the cap (40) is includes a substantially resilient material at its surfaces which are in contact with the swabbable portion (21) at the first state.

9. The assembly according to any one of the claims 1 to 8, further comprising a holder (30) adapted to provide said releasable connection between the needle hub (12) and the main body (10), by having a first end secured to or integral with the needle hub (12) and a second end secured to or integral with the main body; the holder (30) is adapted to break when bent or forced along or around the retraction axis (X).

10. The assembly according to the claim 9, wherein the holder (30) includes a weakened portion (31) adapted to rupture in case where the holder (30) is bent.

11. The assembly according to the claim 10, wherein the holder (30) is formed from a substantially inflexible material.

12. The assembly according to the claim 11, wherein said inflexible material includes a thermoset resin.

13. The assembly according to any one of the claims 9 to 12, wherein said weakened portion (31) is located at said second end of the holder (30), or in vicinity of said second end of the holder (30).

## Patentansprüche

1. Anordnung eines Gefäßzugangskatheters (1), umfassend:
- einen Hauptkörper (10),
- eine Nadel (11), die an einem Nadelansatz (12) befestigt ist, der lösbar mit dem Hauptkörper (10) verbunden ist,
- eine Kanüle (50), die an dem Hauptkörper (10) angebracht ist und so angepasst ist, dass sie die Nadel (11) koaxial umgibt, und
- ein abwischbares intravenöses Ventil (20), das fest an dem Hauptkörper (10) angebracht ist, so dass das intravenöse Ventil (20) in einem ersten Zustand der Anordnung (1) im Wesentlichen um eine Rückzugsachse (X) der Nadel (11) positioniert ist;
**dadurch gekennzeichnet**
- das Ventil (20) einen Kern (22) umfasst;
wobei
die Anordnung eine Kappe (40) umfasst, die lösbar mit dem Hauptkörper (10) verbunden ist; und der Nadelansatz (12) ist fest an der Kappe (40) angebracht oder mit der Kappe (40) integriert;
das Ventil (20) durch seine Form, Größe und Position relativ zum Hauptkörper (10) so angepasst ist, dass es, beim Bringen die Anordnung (1) von ihrem ersten Zustand zu einem zweiten Zustand, relativ zum Hauptkörper (10) entlang der Rückzugachse (X) in Richtung der Kanüle (50) beweglich ist, bis eine Spitze der Nadel (11) aus der Kanüle (50) exponiert wird; und
das Ventil (20) einen Spalt (23) zwischen dem Kern (22) und der Kappe (40) aufweist, der dazu geeignet ist, durch Bewegen der Kappe (40) zusammen mit dem Ventil (20) in Richtung des Kerns (22) gefüllt zu werden, wenn die Anordnung von dem ersten Zustand in den zweiten Zustand gebracht wird.

2. Anordnung nach Anspruch 1, wobei die Kappe (40) eine Größe und Form hat, um ein Ende der Nadelansatz (12) distal zur Kanüle (10) zumindest teilweise zu bedecken.

3. Anordnung nach einem der Ansprüche 1 oder 2, wobei die Kappe (40) über eine manipulationssichere Verbindung mit dem Hauptkörper (10) verbunden ist.

4. Anordnung nach einem der Ansprüche 1 bis 3, wobei die Kappe (40) und der Hauptkörper (10) Schraubgewinde aufweisen, die dafür ausgelegt sind, lösbar miteinander in Eingriff zu kommen.

5. Anordnung nach Anspruch 4, wobei die Kappe (40) durch ihre Form und Größe so angeordnet ist, dass sie ferner einen abwischbaren Abschnitt (21) des Ventils (20) in einem Hohlraum (42) davon aufnimmt, so dass der abwischbare Abschnitt (21) im ersten Zustand der Anordnung (1) im Wesentlichen von der Umgebung isoliert ist.

6. Anordnung nach Anspruch 5, wobei der Hohlraum (42) der Kappe (40) ein Reinigungsmittel enthält, das eine Reinigungsflüssigkeit umfasst, die zum Reinigen oder Sterilisieren der abwischbaren intravenösen Ventile geeignet ist.

7. Anordnung nach Anspruch 6, wobei der Nadelansatz (12) an dem Hohlraum (42) der Kappe (40) befestigt ist.

8. Anordnung nach einem der Ansprüche 1 bis 7, wobei die Kappe (40) an ihren Oberflächen, die im ersten Zustand mit dem abwischbaren Abschnitt (21) in Kontakt sind, ein im Wesentlichen elastisches Material aufweist.

9. Anordnung nach einem der Ansprüche 1 bis 8, die ferner einen Halter (30) umfasst, der so beschaffen ist, dass er die lösbare Verbindung zwischen dem Nadelansatz (12) und dem Hauptkörper (10) bereitstellt, indem er ein erstes Ende aufweist, das an der Nadelansatz (12) befestigt oder mit dieser integriert ist, und ein zweites Ende, das an dem Hauptkörper befestigt oder mit diesem integriert ist; der Halter (30) so beschaffen ist, dass er bricht, wenn er entlang oder um die Rückzugsachse (X) gebogen oder gezwungen wird.

10. Anordnung nach Anspruch 9, wobei der Halter (30) einen geschwächten Abschnitt (31) umfasst, der dafür ausgelegt ist, zu brechen, falls der Halter (30) gebogen wird.

11. Anordnung nach Anspruch 10, wobei der Halter (30) aus einem im Wesentlichen unflexiblen Material gebildet ist.

12. Anordnung nach Anspruch 11, wobei das unflexible Material ein duroplastisches Harz enthält.

13. Anordnung nach einem der Ansprüche 9 bis 12, wobei der geschwächte Abschnitt (31) an dem zweiten Ende des Halters (30) oder in der Nähe des zweiten Endes des Halters (30) angeordnet ist.

## Revendications

1. Ensemble (1) de cathéter d'accès vasculaire comprenant :
- un corps principal (10),
- une aiguille (11) fixée à un moyeu d'aiguille (12) qui est relié de manière amovible au corps principal (10),
- une canule (50) qui est fixée au corps principal (10) et adaptée pour entourer l'aiguille (11) de manière coaxiale, et
- une valve intraveineuse essuyable (20) solidement fixée au corps principal (10), de sorte qu'à un premier état de l'ensemble (1), la valve intraveineuse (20) est positionnée sensiblement autour d'un axe de rétraction (X) de l'aiguille (11);
**caractérisé par**
- la valve (20) comprenant un noyau (22);
dans lequel
l'ensemble comprend un capuchon (40) qui est relié de manière amovible au corps principal (10), et le moyeu d'aiguille (12) est fixé au capuchon (40), ou fait partie intégrante du capuchon (40);
la valve (20) est adaptée par sa forme, sa taille et sa position par rapport au corps principal (10), pour être mobile par rapport au corps principal (10) le long de l'axe de rétraction (X) vers la canule (50) en amenant l'ensemble (1) de son premier état à un deuxième état en déplaçant le moyeu d'aiguille (12) vers le corps principal (10) jusqu'à ce qu'une pointe de l'aiguille (11) soit exposée depuis la canule (50); et
la valve (20) comprend un espace (23) entre le noyau (22) et le capuchon (40) adapté pour être rempli en déplaçant le capuchon (40) avec la valve (20) vers le noyau (22) en amenant l'ensemble du premier état au deuxième état.

2. Ensemble selon la revendication 1, dans lequel le capuchon (40) a une taille et une forme pour recouvrir au moins partiellement une extrémité du moyeu d'aiguille (12) distale par rapport à la canule (10).

3. Ensemble selon l'une quelconque des revendications 1 ou 2, dans lequel le capuchon (40) est relié au corps principal (10) par une connexion inviolable.

4. Ensemble selon l'une quelconque des revendications 1 à 3, dans lequel le capuchon (40) et le corps principal (10) ont des filets de vis adaptés pour s'engager de manière amovible l'un avec l'autre.

5. Ensemble selon la revendication 4, dans lequel le capuchon (40) est agencé par sa forme et sa taille pour recevoir en outre une partie essuyable (21) de la valve (20) dans une cavité (42) de celle-ci, de sorte que ladite partie essuyable (21) est sensiblement isolé de l'environnement dans le premier état de l'ensemble (1).

6. Ensemble selon la revendication 5, dans lequel ladite cavité (42) du capuchon (40) comprend un moyen de nettoyage comprenant un liquide de nettoyage qui convient au nettoyage ou à la stérilisation des valves intraveineuses essuyables.

7. Ensemble selon la revendication 6, dans lequel le moyeu d'aiguille (12) est fixé à la cavité (42) du capuchon (40).

8. Ensemble selon l'une quelconque des revendications 1 à 7, dans lequel le capuchon (40) comprend un matériau sensiblement résilient au niveau de ses surfaces qui sont en contact avec la partie essuyable (21) au premier état.

9. Ensemble selon l'une quelconque des revendications 1 à 8, comprenant en outre un support (30) adapté pour fournir ladite connexion amovible entre le moyeu d'aiguille (12) et le corps principal (10), en ayant une première extrémité fixée à ou intégrée avec le moyeu d'aiguille (12) et une deuxième extrémité fixée ou intégrée au corps principal; le support (30) est adapté pour se casser lorsqu'il est plié ou forcé le long ou autour de l'axe de rétraction (X).

10. Ensemble selon la revendication 9, dans lequel le support (30) comprend une partie affaiblie (31) adaptée pour se rompre dans le cas où le support (30) est plié.

11. Ensemble selon la revendication 10, dans lequel le support (30) est formé d'un matériau sensiblement rigide.

12. Ensemble selon la revendication 11, dans lequel ledit matériau rigide comprend une résine thermodurcissable.

13. Ensemble selon l'une quelconque des revendications 9 à 12, dans lequel ladite partie affaiblie (31) est située au niveau de ladite deuxième extrémité du support (30), ou à proximité de ladite deuxième extrémité du support (30).
